# EUROPEAN PATENT SPECIFICATION

(11) **EP 4 360 692 B1**
(45) Date of publication and mention of the grant of the patent: **20.08.2025**
(21) Application number: 23206258.8
(22) Date of filing: 26.10.2023
(51) Int. Cl.: A61N 1/32, A61N 1/36, A61N 5/06, A61N 5/067, A61N 7/00, A61H 23/02

(54) **ELECTRONIC APPARATUS FOR THE TREATMENT OF BODY IMPERFECTIONS**
ELEKTRONISCHES GERÄT ZUR BEHANDLUNG VON KÖRPERFEHLERN
APPAREIL ÉLECTRONIQUE POUR LE TRAITEMENT D'IMPERFECTIONS CORPORELLES

(30) Priority: 27.10.2022 IT 202200022128; 21.11.2022 DE 202022106495 U; 27.12.2022 AT 5019322 U
(43) Date of publication of application: 01.05.2024
(73) Proprietor: Quaranta, Cesare, 10025 Pino Torinese (TO) (IT)
(72) Inventor: Quaranta, Cesare, 10025 Pino Torinese (TO) (IT)
(74) Representative: Modiano, Micaela Nadia

(56) References cited:
- EP-A1- 3 320 951
- WO-A1-2011/077466
- US-A1- 2014 276 247
- US-A1- 2015 217 142
- US-A1- 2021 020 825
- US-A1- 2022 169 497
- US-B2- 9 492 686

## Description

The present invention relates to an electronic apparatus for the treatment of body imperfections.

In the sector of beauty treatments, among the technologies most used nowadays, electro stimulation, ultrasound, and laser radiation take on particular importance.

Each one of the aforementioned technologies brings specific benefits relating to the reduction of body imperfections.

In particular, electrostimulation works via the emission of electric pulses, through pairs of electrodes which are applied on the body and are subjected to voltages with modulated waveforms at a frequency that can generally vary between 1 and 5000 Hz. Such action induces muscle and tissue toning which improves the oxygenation and revitalization of the tissues, so favoring metabolic exchange and the elimination of toxins.

Ultrasound on the other hand produces a mainly massaging effect. In particular, as is known, ultrasound is efficiently transmitted by the stagnant liquids of the body, making them vibrate and as a consequence inducing a draining effect. Furthermore, the impact of ultrasound on human tissue produces a localized metabolic effect by virtue of the dissipation of heat. The multiplicity of vibrations increases the permeability of the cellular membrane, favoring extracellular exchanges so as to improve the physiological conditions of the cell. Overall, the action of ultrasound makes it possible to remodel localized adipose deposits, to render tissues smoother and more compact, so combating blemishes such as "orange-peel" skin.

Laser treatments are performed by irradiating chosen regions of the body with laser beams that have a preset wavelength depending on the type of treatment to be carried out and, as a consequence, on the desired effect, which can be to mobilize adipose deposits and firm the surrounding skin tissue, or a toning effect, or other correlated effects.

Although it is known to combine the action of electro stimulation with that of ultrasound and laser in a single device to be applied to the user being treated, thus far difficulties have been encountered with maximizing the effectiveness of the three above-mentioned treatments in the known combined apparatuses.

Such difficulties are due to the fact that the three technologies mentioned above require specific technical contrivances which in some cases can be in conflict with each other, or have side effects that can compromise the reliability of the system, as in the case of the heating caused by the ultrasound returning to the electronic components inside the device. With particular regard to this latter aspect, as is well known to the person skilled in the art, the risk of overheating is one of the factors that currently limits the duty cycle of the square wave normally used to produce ultrasound. As a consequence, in the current applications, the duty cycle usually does not exceed 50%, even if it is actually desirable to increase it in order to increase the efficacy of the treatment. US 2014/276247 A1 discloses a device for delivering light and ultrasound across a skin surface, wherein the device has a layered structure comprising a light source and an ultrasonic transducer.

EP 3 320 951 A1 discloses an applicator hand-piece used for therapeutic and cosmetic treatments, has head that has piezoelectric transducer and shaped plates for receiving electrical signals used to generate shock waves and diathermic currents for zone to be treated.

The aim of the present invention therefore is to provide an electronic apparatus for the treatment of body imperfections that is capable of combining, in a single device, the treatments of electrostimulation, ultrasound and laser irradiation, more effectively and reliably than in known solutions.

The above aim and other objects, which will become clearer from the description that follows, are achieved by an electronic apparatus having the characteristics recited in the appended claim 1, while the appended dependent claims define other characteristics of the invention which are advantageous, although secondary.

Now the invention will be described in greater detail, with reference to a preferred but not exclusive embodiment thereof, which is illustrated for the purposes of non-limiting example in the accompanying drawings, wherein:
- Figure 1 is an exploded perspective view of a component of the electronic apparatus according to the invention;
- Figure 2 is a side view of the electronic apparatus of Figure 1;
- Figure 3 is a cross-sectional view of Figure 2 taken along the line III-III;

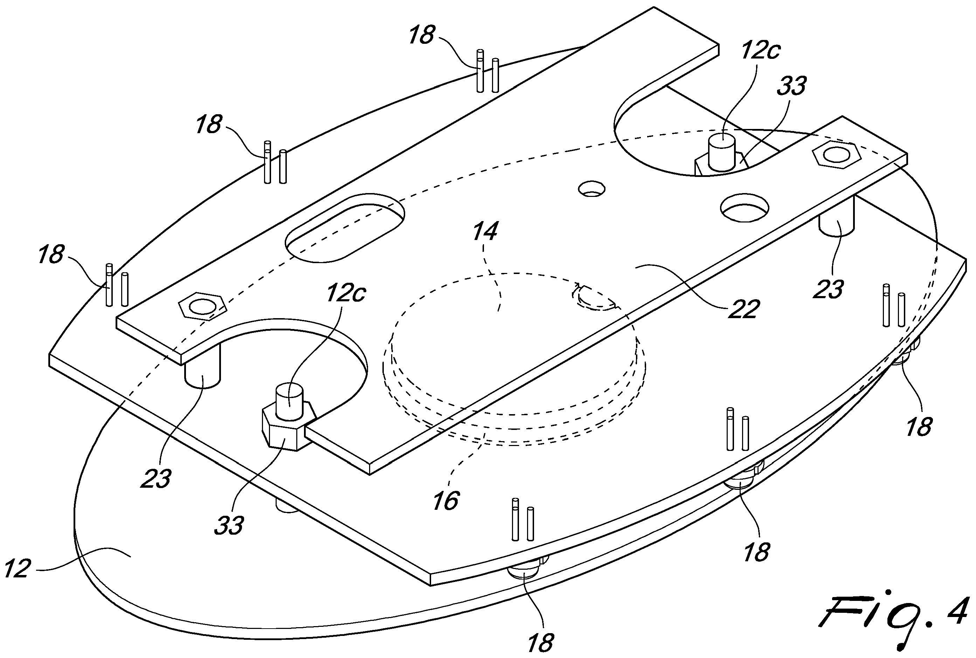

- Figure 4 is a perspective view showing only some elements of the component of Figure 1 in the assembled configuration;
- Figure 5 is a block diagram of the electronic apparatus according to the invention.

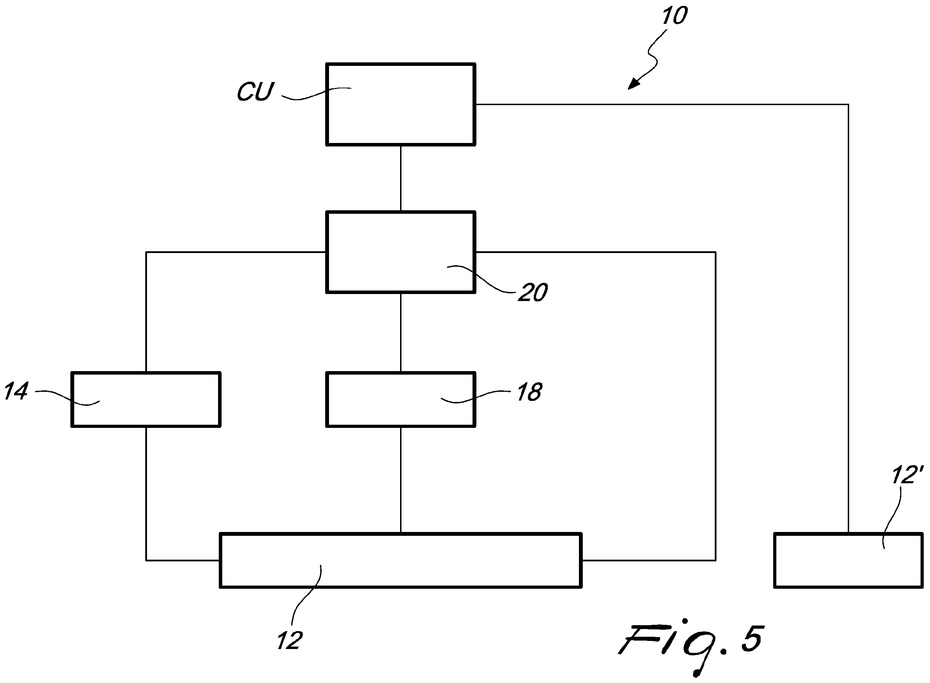

With reference to the figures, an electronic apparatus for the treatment of body imperfections, generally designated by the reference numeral 10 in the block diagram in Figure 5, comprises at least one device 11 which is provided with:
- a plate-like electrode 12 made of an electrically conductive material, for example metal, which is provided with a contact surface 12a adapted to come into contact with the body of a user subjected to the treatment, and is subjected to a voltage with a modulated waveform, preferably at an adjustable frequency comprised between 1 and 5000 Hz, for generating controlled electrical pulses by interacting with a closing electrode (generally designated by the reference numeral 12' in Fig. 5) which is also applied to the body of the user, preferably but not necessarily the plate-like electrode of a second, identical device,
- a piezoelectric transducer 14, preferably made of ceramic material, which is fixed to the plate-like electrode 12 on the opposite side with respect to the contact surface 12a and is subjected to a voltage with a modulated waveform, preferably at an adjustable frequency comprised between 3.0 and 3.6 Mhz, for generating ultrasound,
- means of electrical insulation interposed between the plate-like electrode 12 and the piezoelectric transducer 14, preferably a sheet of mica 16,
- a plurality of laser sources 18 arranged externally to the perimeter of the plate-like electrode 12 and subjected to a voltage with a modulated waveform for emitting respective laser beams having a predetermined wavelength depending on the type of laser treatment to be performed,
- an electronic board 20, to which the plate-like electrode 12, the piezoelectric transducer 14 and the laser sources 18 are connected.

The electronic board 20 is connected to a programmable control unit CU (only shown schematically in the block diagram of Figure 5) in order to vary the parameters for driving at least one among the plate-like electrode 12, the piezoelectric transducer 14 and the laser sources 18.

The laser sources 18 can be conventional diodes and, advantageously, the device 11 comprises six such diodes. In this embodiment, in which the plate-like electrode 12 has an oval profile, the six laser sources 18 are arranged in two groups of three along the mutually opposite long sides of the oval.

With reference to Figure 5, in the electronic apparatus 10 according to the invention the laser sources 18 (depicted overall by a single block in Figure 5) are connected in series to each other and in parallel with the piezoelectric transducer 14, so that the laser sources 18 and the piezoelectric transducer 14 are driven by the same voltage signal. Advantageously, the characteristics of the voltage signal, in particular the frequency, the duty cycle and the peak voltage, are defined by the control unit CU, with the possibility for the user to adjust the duty cycle.

In a preferred embodiment of the invention, the piezoelectric transducer 14 is subjected to a voltage having a square waveform with a duty cycle equal or proximate to 100%, preferably at least above 75%, and, in order to forestall the risk of overheating of the internal components of the device 11, particularly of the electronic board 20, owing to the return of the ultrasound, the device 11 is provided with a heat sink 22 which is associated with the electronic board 20.

According to a preferred embodiment, the electronic board 20 is supported parallel to the plate-like electrode 12 above the piezoelectric transducer 14, and the heat sink 22 comprises a metallic lamina which in turn is supported above, and parallel to, the electronic board 20 in a position that is spaced apart from the latter, preferably by means of a pair of spacer columns 23. It should be understood that in the present description and in the claims, terms like "above" or "below" should be understood to take the contact surface 12a of the plate-like electrode 12 as the lower reference point.

The plate-like electrode 12 receives a signal that arrives from the control unit CU and is filtered through the electronic board 20. The waveforms that produce the electrostimulation can be square or sinusoidal, and the effect obtained (draining, reduction of cellulite blemishes, and other possible correlated effects) derives from the carrier frequency, which can be further modulated at other frequencies.

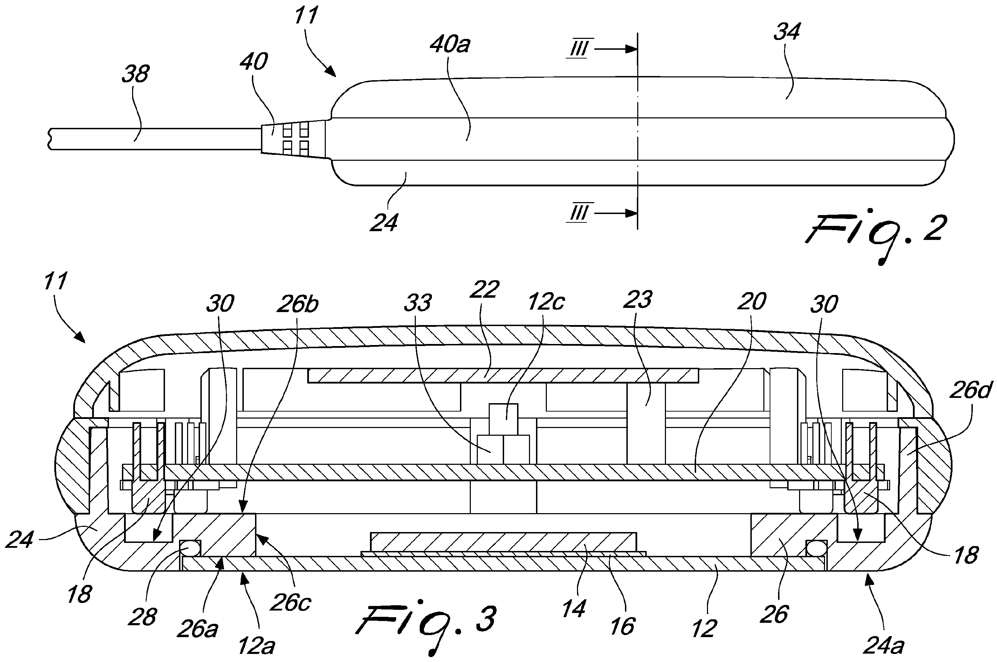

The device 11 is provided with a frame 24 made of transparent material, preferably plastic material such as ABS, which has a flat base 26. The plate-like electrode 12 rests parallel on a lower surface 26a (Figure 3) of the flat base 26, advantageously with the interposition of an annular gasket 28. The laser sources 18 are soldered to the electronic board 20 and facing an upper surface 26b of the flat base 26, preferably at respective calibrated recesses 30, so that the respective laser beams are irradiated through the transparent material of the frame 24 from a lower flat annular surface 24a (Figure 3) of the latter, which surrounds the contact surface 12a of the plate-like electrode 12. Advantageously, the lower flat annular surface 24a is substantially coplanar with the contact surface 12a.

The flat base 26 also has a central opening 26c which surrounds the piezoelectric transducer 14.

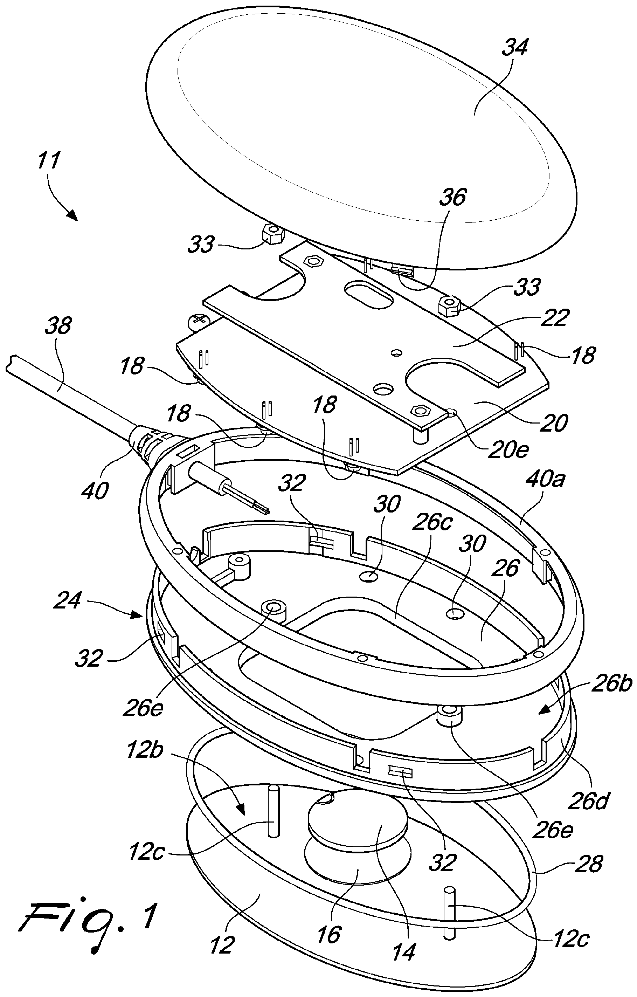

A perimeter wall 26d provided with engagement openings 32 (Figure 1) rises from the outer edge of the flat base 26.

A pair of threaded pegs 12c rise perpendicularly from the upper face 12b of the plate-like electrode 12, opposite to the contact surface 12a. The threaded pegs 12c are inserted into respective first through holes 26e of the flat base 26 and second through holes 20e of the electronic board 20 (Figure 1). The frame 24 is sandwiched between the plate-like electrode 12 and the electronic board 20 and tightened with a pair of nuts 33 screwed onto the threaded pegs 12c above the electronic board 20.

The device 11 is provided with a cover 34 molded from plastic material, which is adapted to be mounted on the frame 24 and, to this end, is provided with teeth 36 (Figure 1) which engage by snap action in the engagement openings 32.

Advantageously, the control unit CU is not incorporated in the device 11 but is located in a remote position, in a manner that is per se known.

The device 11 is connected to the control unit CU by a wire 38 inserted into a gland 40 which has an annular support portion 40a which, in the assembled configuration, encircles the perimeter wall 26d and remains sandwiched between the frame 24 and the cover 34.

When the cover 34 is applied, advantageously the contact surface 12a and the lower flat annular surface 24e of the frame 24 remain in view.

Typically, during a session a plurality of devices 11 of the type described above can be applied simultaneously on the body of the user, e.g., sixteen or twenty-four devices distributed in pairs, each one of which is capable of simultaneously executing electrostimulation, ultrasound and laser irradiation treatments.

In particular, the plate-like electrode 12, being placed against the piezoelectric transducer 14 with the interposition of only the sheet of mica 16, ensures a high transmission of the acoustic wave of the ultrasound. To the foregoing is added the action of the laser sources 18 which, by virtue of their specific arrangement around the plate-like electrode 12 described previously, can simultaneously irradiate the same regions of the body that are already subjected to electrostimulation and to ultrasound. As the person skilled in the art will be able to appreciate, the action of the laser sources 18 arranged around the plate-like electrode 12 does not interfere with the electrostimulation and the ultrasound.

By virtue of the connection in parallel of the laser sources 18 (which are connected to each other in series) with the piezoelectric transducer 14, the important advantage is obtained of using the same electric voltage to power different means of generation of output (in the example described herein, a piezoelectric transducer and six laser diodes) and obtain combined effects on the human body.

At the same time, the heating caused by the returning ultrasound, which is particularly high owing to the duty cycle substantially equal to 100% of the square wave that drives the ultrasound and the laser, is effectively dissipated by the heat sink 22, so as not to compromise the performance and reliability of the system.

A preferred embodiment of the invention has been described, but obviously the person skilled in the art may make various modifications and variations within the scope of protection of the claims.

For example, the plate-like electrode 12 and the frame 24 can have different profiles from the oval profiles shown here for the purposes of example, e.g., circular profiles, rectangular profiles etc.

Furthermore it should be noted that, only for electrostimulation, in order to close the circuit it would be sufficient to associate the device 11 with an electrode of conventional type provided with its own electronics (generally designated by 12' in Figure 5), although it is preferable to use a second, identical device in order to maximize the efficacy of the treatment.

The lower perimeter surface 24a could be slightly retracted instead of being coplanar with respect to the contact surface 12a.

It is also understood that the electronic apparatus described herein, although it is predominantly conceived for beauty treatments, could also be applied in other sectors, in particular in the electromedical sector.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An electronic apparatus for the treatment of body imperfections, comprising at least one device (11) provided with:
- a plate-like electrode (12) made of an electrically conductive material, which is provided with a contact surface (12a) adapted to come into contact with the body of a user subjected to the treatment, and is subjected to a voltage with a modulated waveform for generating controlled electrical pulses by interacting with a closing electrode (12') which is also applied to the body of the user,
- a piezoelectric transducer (14) which is fixed to said plate-like electrode (12) on the opposite side with respect to said contact surface (12a) and is subjected to a voltage with a modulated waveform for generating ultrasound,
- means of electrical insulation (16) interposed between said plate-like electrode (12) and said piezoelectric transducer (14),
- a plurality of laser sources (18) subjected to a voltage with a modulated waveform for emitting respective laser beams having a predetermined wavelength depending on the type of laser treatment to be performed, and
- an electronic board (20), to which said plate-like electrode (12), said piezoelectric transducer (14) and said laser sources (18) are connected,
said electronic board (20) being connected to a control unit (CU) which is programmable to vary the driving parameters of at least one among said plate-like electrode (12), said piezoelectric transducer (14) and said laser sources (18), **characterized in that** said laser sources (18) are arranged externally to the perimeter of said plate-like electrode (12) at a position that does not interfere with the electro stimulation and the ultrasound, and are connected in series to each other and in parallel with said piezoelectric transducer (14), so that said laser sources (18) and said piezoelectric transducer (14) are driven by a same voltage signal.

2. The electronic apparatus (10) according to claim 1, **characterized in that** the frequency, the duty cycle and the peak voltage of said voltage signal are defined by said control unit (CU), with the possibility for a user to adjust the duty cycle.

3. The electronic apparatus (10) according to claim 1 or 2, **characterized in that** said piezoelectric transducer (14) is subjected to a voltage having a square waveform with a duty cycle greater than 75%, and **in that** it comprises a heat sink (22) associated with said electronic board (20).

4. The electronic apparatus (10) according to claim 3, **characterized in that** said electronic board (20) is supported parallel to said plate-like electrode (12) above said piezoelectric transducer (14), and **in that** said heat sink (22) comprises a metallic lamina which is supported above, and parallel to, said electronic board (20) in a position that is spaced apart from the latter.

5. The electronic apparatus (10) according to claim 3 or 4, **characterized in that** said square waveform has a frequency comprised between 3.0 and 3.6 MHz.

6. The electronic apparatus (10) according to one of claims 1-5, **characterized in that** it comprises a frame (24) made of a transparent material provided with a flat base (26), said plate-like electrode (12) resting in parallel on a lower surface (26a) of said flat base (26), said laser sources (18) being soldered to said electronic board (20) and facing an upper surface (26b) of the flat base (26), so that the respective laser beams are irradiated through the transparent material of said frame (24), from a lower flat annular surface (24a) of the latter, which surrounds said contact surface (12a).

7. The electronic apparatus (10) according to claim 6, **characterized in that** said lower flat annular surface (24a) is substantially coplanar with said contact surface (12a).

8. The electronic apparatus (10) according to claim 6 or 7, **characterized in that** it comprises a cover (34) molded from plastic material which engages said frame (24) so as to leave said contact surface (12a) and said lower flat annular surface (24a) exposed.

9. The electronic apparatus (10) according to one of claims 1-8, **characterized in that** said control unit (CU) is located remotely with respect to said device (11).

10. The electronic apparatus (10) according to one of claims 1-9, **characterized in that** said means of electrical insulation comprise a sheet of mica (16).

## Patentansprüche

1. Eine elektronische Vorrichtung zur Behandlung körperlicher Makel, die mindestens eine Vorrichtung (11) umfasst, ausgestattet mit
- einer plättchenartigen Elektrode (12) aus einem elektrisch leitenden Material, die mit einer Kontaktfläche (12a) ausgestattet ist, ausgebildet, um in Kontakt mit dem Körper eines Benutzers zu kommen, der der Behandlung unterzogen wird, und einer Spannung mit modulierter Wellenform zur Erzeugung gesteuerter elektrischer Impulse ausgesetzt wird durch Interagieren mit einer Schließelektrode (12'), die ebenfalls an den Körper des Benutzers angebracht wird,
- einen piezoelektrischen Wandler (14), der an der plättchenartigen Elektrode (12) an der Seite angebracht ist, die der Kontaktfläche (12a) gegenüberliegt, und einer Spannung mit modulierter Wellenform zur Erzeugung von Ultraschall ausgesetzt wird,
- Mittel zur elektrischen Isolierung (16), angeordnet zwischen der plättchenartigen Elektrode (12) und dem piezoelektrischen Wandler (14),
- eine Vielzahl von Laserquellen (18), die einer Spannung mit modulierter Wellenform ausgesetzt sind zum Senden entsprechender Laserstrahlen mit einer vordefinierten Wellenlänge abhängig von der Art der durchzuführenden Laserbehandlung, und
- eine Leiterplatte (20), an welche die plättchenartige Elektrode (12), der piezoelektrische Wandler (14) und die Laserquellen (18) angeschlossen sind,
wobei die Leiterplatte (20) eine Steuereinheit (control unit, CU) angeschlossen ist, die programmierbar ist, um die Antriebsparameter mindestens eines der plättchenartigen Elektrode (12), des piezoelektrischen Wandlers (14) und der Laserquellen (18) zu verändern; **dadurch gekennzeichnet, dass** die Laserquellen (18) außerhalb des Umfangs der plättchenartigen Elektrode (12) in einer Position angeordnet sind, welche die Elektrostimulation und den Ultraschall nicht behindert, und seriell zueinander und parallel zu dem piezoelektrischen Wandler (14) angeschlossen sind, so dass die Laserquellen (18) und der piezoelektrische Wandler (14) von einem selben Spannungssignal betrieben werden.

2. Die elektronische Vorrichtung (10) gemäß Anspruch 1, **dadurch gekennzeichnet, dass** die Frequenz, der Tastgrad und die Spitzenspannung den Spannungssignals von der Steuereinheit (CU) bestimmt werden, mit der Möglichkeit, dass ein Benutzer den Tastgrad anpasst.

3. Die elektronische Vorrichtung (10) gemäß Anspruch 1 oder 2, **dadurch gekennzeichnet, dass** der piezoelektrische Wandler (14) einer Spannung ausgesetzt ist, die eine quadratische Wellenform hat, mit einem Tastgrad über 75%, und dadurch, dass er eine Wärmesenke (22) umfasst, die mit der Leiterplatte (20) verbunden ist.

4. Die elektronische Vorrichtung (10) gemäß Anspruch 3, **dadurch gekennzeichnet, dass** die Leiterplatte (20) parallel zu der plättchenartigen Elektrode (12) oberhalb des piezoelektrischen Wandlers (14) gelagert ist, und dadurch, dass die Wärmesenke (22) ein Metallplättchen umfasst, das oberhalb und parallel zu der Leiterplatte (20) in einer Position gelagert ist, die von Letzterer beabstandet ist.

5. Die elektronische Vorrichtung (10) gemäß Anspruch 3 oder 4, **dadurch gekennzeichnet, dass** die quadratische Wellenform eine Frequenz zwischen 3,0 und 3,6 MHz hat.

6. Die elektronische Vorrichtung (10) gemäß einem der Ansprüche 1-5, **dadurch gekennzeichnet, dass** sie einen Rahmen (24) aus einem transparenten Material umfasst, ausgestattet mit einer flachen Basis (26), wobei die plättchenartige Elektrode (12) parallel auf einer unteren Oberfläche (26a) der flachen Basis (26) aufliegt, wobei die Laserquellen (18) an die Leiterplatte (20) angelötet und einer oberen Oberfläche (26b) der flachen Basis (26) zugewandt sind, so dass die jeweiligen Laserstrahlen von einer unteren, flachen, ringförmigen Fläche des Rahmens, die die Kontaktfläche (12a) umgibt, durch das transparente Material des Rahmens (24) geleitet werden.

7. Die elektronische Vorrichtung (10) gemäß Anspruch 6, **dadurch gekennzeichnet, dass** die untere, flache, ringförmige Fläche (24a) im Wesentlichen koplanar mit der Kontaktfläche (12a) ist.

8. Die elektronische Vorrichtung (10) gemäß Anspruch 6 oder 7, **dadurch gekennzeichnet, dass** sie eine Abdeckung (34) umfasst, die aus Kunststoffmaterial geformt ist und den Rahmen (24) so umgibt, dass die Kontaktfläche (12a) und die untere, flache, ringförmige Oberfläche (24a) frei bleiben.

9. Die elektronische Vorrichtung (10) gemäß einem der Ansprüche 1-8, **dadurch gekennzeichnet, dass** die Steuereinheit (CU) entfernt von der Vorrichtung (11) positioniert ist.

10. Die elektronische Vorrichtung (10) gemäß einem der Ansprüche 1-9, **dadurch gekennzeichnet, dass** die Mittel zur elektrischen Isolierung eine Schicht aus Glimmermineral (16) umfassen.

## Revendications

1. Un appareil électronique destiné au traitement des imperfections corporelles, comprenant au moins un dispositif (11) muni de :
- une électrode en forme de plaque (12) réalisée en un matériau électriquement conducteur, qui est pourvue d'une surface de contact (12a) adaptée pour venir en contact avec le corps d'un utilisateur soumis au traitement, et qui est soumise à une tension avec une forme d'onde modulée pour générer des impulsions électriques contrôlées en interagissant avec une électrode de fermeture (12') qui est également appliquée sur le corps de l'utilisateur,
- un transducteur piézoélectrique (14) qui est fixé à ladite électrode en forme de plaque (12) sur le côté opposé par rapport à ladite surface de contact (12a) et est soumis à une tension avec une forme d'onde modulée pour générer des ultrasons,
- des moyens d'isolation électrique (16) interposés entre ladite électrode en forme de plaque (12) et ledit transducteur piézoélectrique (14),
- une pluralité de sources laser (18) soumises à une tension avec une forme d'onde modulée pour émettre des faisceaux laser respectifs ayant une longueur d'onde prédéterminée en fonction du type de traitement laser à effectuer, et
- une carte électronique (20), à laquelle sont connectées ladite électrode en forme de plaque (12), ledit transducteur piézoélectrique (14) et lesdites sources laser (18),
ladite carte électronique (20) étant connectée à une unité de commande (CU) programmable pour faire varier les paramètres de commande d'au moins un élément parmi ladite électrode en forme de plaque (12), ledit transducteur piézoélectrique (14) et lesdites sources laser (18), **caractérisé en ce que** lesdites sources laser (18) sont disposées à l'extérieur du périmètre de ladite électrode en forme de plaque (12) à une position qui n'interfère pas avec l'électrostimulation et les ultrasons, et sont connectées en série les unes aux autres et en parallèle avec ledit transducteur piézoélectrique (14), de sorte que lesdites sources laser (18) et ledit transducteur piézoélectrique (14) sont commandés par un même signal de tension.

2. L'appareil électronique (10) selon la revendication 1, **caractérisé en ce que** la fréquence, le cycle de service et la tension de crête dudit signal de tension sont définis par ladite unité de commande (CU), avec la possibilité pour un utilisateur d'ajuster le cycle de service.

3. L'appareil électronique (10) selon la revendication 1 ou 2, **caractérisé en ce que** ledit transducteur piézoélectrique (14) est soumis à une tension ayant une forme d'onde carrée avec un cycle de service supérieur à 75 %, et **en ce qu'**il comprend un dissipateur thermique (22) associé à ladite carte électronique (20).

4. L'appareil électronique (10) selon la revendication 3, **caractérisé en ce que** ladite carte électronique (20) est supportée parallèlement à ladite électrode en forme de plaque (12) au-dessus dudit transducteur piézoélectrique (14), et **en ce que** ledit dissipateur thermique (22) comprend une lame métallique qui est supportée au-dessus et parallèlement à ladite carte électronique (20) dans une position espacée de cette dernière.

5. L'appareil électronique (10) selon la revendication 3 ou 4, **caractérisé en ce que** ladite onde carrée a une fréquence comprise entre 3,0 et 3,6 MHz.

6. L'appareil électronique (10) selon l'une des revendications 1 à 5, **caractérisé en ce qu'**il comprend un cadre (24) en matériau transparent muni d'une base plate (26), ladite électrode en forme de plaque (12) reposant parallèlement sur une surface inférieure (26a) de ladite base plate (26), lesdites sources laser (18) étant soudées à ladite carte électronique (20) et faisant face à une surface supérieure (26b) de la base plate (26), de sorte que les faisceaux laser respectifs sont irradiés à travers le matériau transparent dudit cadre (24), à partir d'une surface annulaire plate inférieure (24a) de ce dernier, qui entoure ladite surface de contact (12a).

7. L'appareil électronique (10) selon la revendication 6, **caractérisé en ce que** ladite surface annulaire plane inférieure (24a) est sensiblement coplanaire avec ladite surface de contact (12a).

8. L'appareil électronique (10) selon la revendication 6 ou 7, **caractérisé en ce qu'**il comprend un couvercle (34) moulé en matière plastique qui s'engage dans ledit cadre (24) de manière à laisser exposées ladite surface de contact (12a) et ladite surface annulaire plane inférieure (24a).

9. L'appareil électronique (10) selon l'une des revendications 1 à 8, **caractérisé en ce que** ladite unité de commande (CU) est située à distance dudit dispositif (11).

10. L'appareil électronique (10) selon l'une des revendications 1 à 9, **caractérisé en ce que** lesdits moyens d'isolation électrique comprennent une feuille de mica (16).
